# NEW EUROPEAN PATENT SPECIFICATION

(11) **EP 1 374 770 B2**
(45) Date of publication and mention of the opposition decision: **10.08.2011**
(45) Mention of the grant of the patent: 28.11.2007
(21) Application number: 03014233.5
(22) Date of filing: 25.06.2003
(51) Int. Cl.: A61B 5/15, A61B 10/00

(54) **Sensor with integrated lancet**
Sensor mit integrierter Lanzette
Capteur à lancette intégrée

(30) Priority: 25.06.2002 US 391108 P
(43) Date of publication of application: 02.01.2004
(60) Divisional application: 07013176.8
(73) Proprietor: Bayer HealthCare LLC, Tarrytown, NY 10591 (US)
(72) Inventor: Vreeke, Mark S., Houston, Texas 77070 (US); Charlton, Steven C., Osceola, Indiana 46561 (US); McCleary, Alan R., South Bend, Indiana 46637 (US); Flora, Bruce A., Goshen, Indiana 46528 (US)
(74) Representative: Linhart, Angela

(56) References cited:
- EP-A- 1 174 083
- WO-A-01/72220
- WO-A1-97/00441
- JP-A- 258 382
- US-A- 4 627 445
- US-A- 5 879 311
- US-A- 5 971 941
- US-A- 6 132 449

## Description

### FIELD OF THE INVENTION

The present invention relates generally to blood monitoring devices and, more particularly, to a sensor having an integrated lance.

### BACKGROUND OF THE INVENTION

It is often necessary to quickly obtain a sample of blood and perform an analysis of the blood sample. One example of a need for quickly obtaining a sample of blood is in connection with a blood glucose monitoring system where a user must frequently use the system to monitor the user's blood glucose level.

Those who have irregular blood glucose concentration levels are often medically required to self-monitor their blood glucose concentration level. An irregular blood glucose level can be brought on by a variety of reasons including illness, such as diabetes. The purpose of monitoring the blood glucose concentration level is to determine the blood glucose concentration level and then to take corrective action, based upon whether the level is too high or too low, to bring the level back within a normal range. The failure to take corrective action can have serious implications. When blood glucose levels drop too low, a condition known as hypoglycemia, a person can become nervous, shaky, and confused.That person's judgment may become impaired and that person may eventually pass out. A person can also become very ill if their blood glucose level becomes too high, a condition known as hyperglycemia. Both conditions, hypoglycemia and hyperglycemia, are potentially life-threatening emergencies.

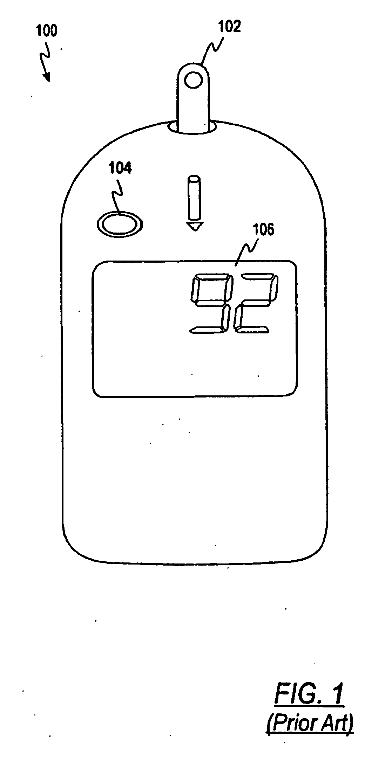

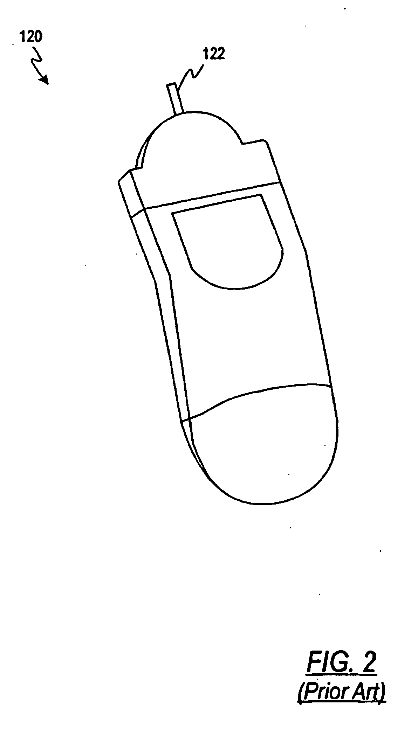

One method of monitoring a person's blood glucose level is with a portable, hand-held blood glucose testing device. A prior art blood glucose testing device 100 is illustrated in FIG. 1. The portable nature of these devices 100 enables the users to conveniently test their blood glucose levels wherever the user may be. The glucose testing device contains a test sensor 102 to harvest the blood for analysis. The device 100 contains a switch 104 to activate the device 100 and a display 106 to display the blood glucose analysis results. In order to check the blood glucose level, a drop of blood is obtained from the fingertip using a lancing device. A prior art lancing device 120 is illustrated in FIG. 2. The lancing device 120 contains a needle lance 122 to puncture the skin. Some lancing devices implement a vacuum to facilitate drawing blood. Once the requisite amount of blood is produced on the fingertip, the blood is harvested using the test sensor 102. The test sensor 102, which is inserted into a testing unit 100, is brought into contact with the blood drop. The test sensor 102 draws the blood to the inside of itself. The test sensor, in combination with the testing unit, then determines the concentration of glucose in the blood. Once the results of the test are displayed on the display 106 of the test device 100, the test sensor 102 is discarded. Each newtest requires a newtest sensor 102.

One problem associated with current test devices is that the test device comprises a two step operation for sample generation and sample harvesting/reading. The two operations are accomplished with two separate instruments (a lance and a test sensor), each having a separate disposable. This requires more parts and more work for the user in disposing the parts.

Another problem associated with current test devices is the difficulty in harvesting small samples when the test sensor is separate from the lance. There is a trend in glucose testing towards minimizing the sample volume. This trend is based on the assumption that there is a corresponding reduction in pain when less sample volume is acquired. As the sample volume is reduced, it becomes more difficult to manually manipulate the test sensor in order to harvest the blood. This is especially true for people who may have seeing impairments or other disabilities, making it difficult to manipulate the test sensor within a small area.

Another problem associated with obtaining small sample sizes is related to the precision needed to obtain the samples. When only small amounts of blood are produced by the lance, it is important that the entire sample or most of the sample be drawn into the test device. When larger volumes of blood are drawn, it is less necessary to obtain all of the blood for the sensor. In small volume test devices, it is important that the sensor be located very near to the puncture wound to maximize the amount of blood that is drawn into the sensor for testing. In current test devices, where the sensor has to be manually moved to the puncture wound, it may be difficult to get close enough to the wound to obtain enough of the sample.

Some current test devices utilize an integrated sensor and lance. The lance is perpendicular to the plane of the test sensor and penetrates through the sensor surface. These sensors, however, still experience the problem that the test sensor must be manually manipulated after the lancing operation is performed.

Another test device has been developed for the collection of interstitial fluid (ISF) that utilizes an integrated lance and reaction area. ISF is collected by piercing just below the skin before any nerve endings or any capillaries. Collecting ISF is sometimes desirable because there is no pain involved since it is above any nerve endings. The lance in this test device is not strong enough to pierce through the dermal layer of the skin in order to obtain samples of other fluids, such as blood. One disadvantage of this and other integrated systems is that the user is forced to dispose of the lance with each test device, an additional expense, as most users reuse their lancets a number of times. A second disadvantage is that any reagent in the device is necessarily exposed to extreme conditions during the required sterilization of the lance. Such exposure may affect the performance of the device.

### SUMMARY OF THE INVENTION

The present invention is a fluid collection apparatus adapted to test a concentration of an analyte in a fluid and includes a lid and a base. The fluid collection apparatus further includes a spacer disposed between the lid and the base. The spacer forms a capillary channel, which has an opening and is designed to collect the fluid. The capillary channel also includes a reagent that reacts with the fluid to produce a measurable reaction. The reaction will indicate the concentration of the analyte in the fluid. A lance is disposed between the lid and the base in the capillary channel. The lance is moveable relative to to the base and is moveable to a position adjacent the opening of the capillary channel.

The above summary of the present invention is not intended to represent each embodiment, or every aspect, of the present invention. This is the purpose of the figures and the detailed description which follow.

### BRIEF DESCRIPTION OF THE DRAWINGS

The foregoing and other advantages of the invention will become apparent upon reading the following detailed description and upon reference to the drawings.

FIG. 3 is a top end view of a test device according to one embodiment of the present invention.

FIG. 4a is a front view of a test device having a cover removed according to one embodiment of the present invention.

FIG. 4b is a front view of a test device having a cover removed according to another embodiment of the present invention.

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within the scope of the invention as defined by the appended claims.

### DESCRIPTION OF ILLUSTRATIVE EMBODIMENTS

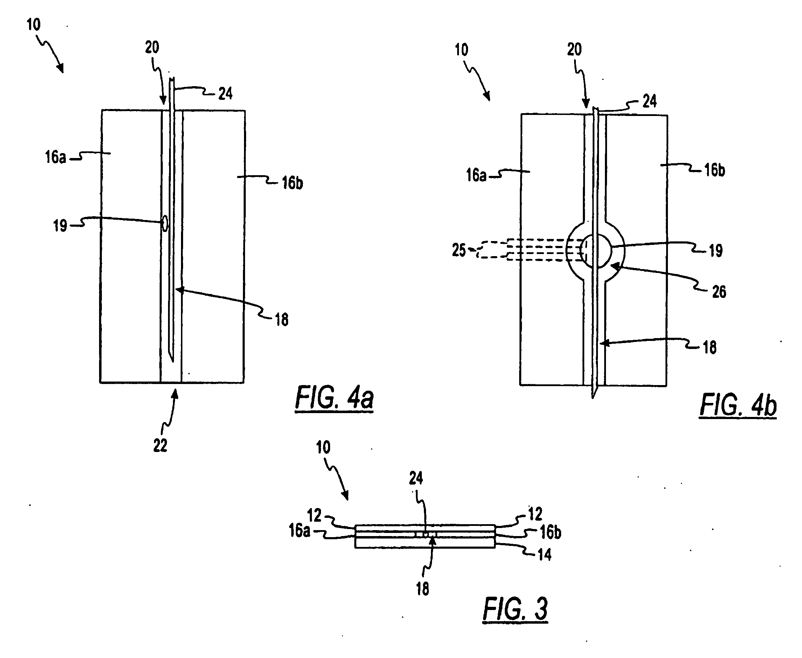

FIG. 3 depicts a fluid collection apparatus 10 according to one embodiment of the present invention. The fluid collection apparatus 10 is designed to collect a fluid, such as blood, so the fluid may be tested for the concentration of a particular analyte, such as glucose. In describing the details of the operation of the fluid collection apparatus 10, the fluid described will be blood pricked from a finger and the analyte will be glucose. It is understood that the embodiment may also be used for other fluids and analytes and that these only serve as examples.

The fluid collection apparatus 10 includes a lid 12, a base 14, and a pair of spacers 16a, 16b disposed between the lid 12 and the base 14. The pair of spacers 16a, 16b form a capillary channel 18. In the illustrated embodiment, the capillary channel 18 is elongated and spans the entire length of the spacers (shown in FIG. 4a). The capillary channel 18 has a first end 20 and a second end 22 (shown in FIG. 4a). The capillary channel 18 includes a reagent 19 that will react with the drawn blood in orderto create a measurable reaction. According to one embodiment, the reagent 19 The capillary channel 18 has a first end 20 and a second end 22 (shown in FIG. 4a). The capillary channel 18 includes a reagent 19 that will react with the drawn blood in order to create a measurable reaction. According to one embodiment, the reagent 19 is disbursed throughout the entire capillary channel. A lance 24 is disposed in the capillary channel 18. The lance 24 is moveable through the capillary channel 18 in a direction parallel to the length of the capillary channel 18.

In one embodiment, the fluid collection apparatus 10 can be used in conjunction with a photometric test device to measure the concentration of the analyte directly, for example, by the absorption of light in the infrared region. The test device would measure the amount of infrared light absorbed. Alternatively, a reagent 19 can be used that causes a change in color in the capillary channel. The photometric test device then reads the amount of color change. Photometric testing is described in more detail in commonly-owned U.S. Patent No. 5,611,999 entitled "Diffuse Reflectance Readhead" which is incorporated herein by reference in its entirety. It is also contemplated that other methods of measuring the concentration of glucose in blood may be utilized.

In another embodiment of the fluid collection apparatus 10, an electrochemical test device is employed as shown in FIG. 4b. The capillary channel 18 includes a pair of electrodes 25. In electrochemical analysis, the change in current across the electrodes 25 caused by the reaction of the glucose and the reagent 19 creates an oxidation current at the electrodes 25 which is directly proportional to the user's blood glucose concentration. The current can be measured by an electrochemical test device coupled to a pair of terminals (not shown) corresponding to the electrodes 25. The electrochemical test device can then communicate to the user the blood glucose concentration. An example of an electrochemical test system is described in detail by commonly-owned U.S. Patent No. 5,723,284 entitled "Control Solution And Method For Testing The Performance Of An Electrochemical Device For Determining The Concentration Of An Analyte In Blood" which is incorporated herein by reference in its entirety.

Turning now to FIG. 4a, a top view of the fluid collection apparatus 10 with the lid 12 removed is shown. As can be seen in this view, the lance 24 extends through the capillary channel 18 and out of the first end 20. The reagent 19 may be placed anywhere within the capillary channel 18.

In FIG. 4b, an alternative embodiment of the fluid collection apparatus 10 is shown. In this embodiment, the capillary channel 18 includes a detection area 26. The detection area 26 may be a reaction area that includes the reagent 19 and is slightly wider than the rest of the capillary channel 18. The enlarged area makes viewing easier and is used with some optical sensors.

In one embodiment, the capillary channel 18 is from approximately 0.51 (0.020) to approximately 1.02 mm (0.040 inches) in length and from approximately 0.15 (0.006) to approximately 0.3 mm (0.012 inches) in width. The lance 24 is from approximately 0.13 (0.005) to approximately 0.28 mm (0.011 inches) in diameter. The detection area 26 has an area of approximately 0.45 (0.7 x 10-³) to approximately 6.45 mm² (10 x 10-³ inches squared).

The operation of the device 10 illustrated in the embodiments of FIGS. 3-4b will now be described. A user will position the apparatus such that the second end 22 of the capillary channel 18 is pressed against the skin. The lance 24 is in a first position, shown in FIG. 4a, extending out from the first end 20 of the capillary channel 18. The user then pushes the lance 24 downward to a second position shown in FIG. 4b, such that the lance 24 extends past the second opening 22 of the capillary channel 18 and enters the skin. The lance 24 is pushed downward with enough force to create a puncture wound sufficient to draw blood. The lance 24 has a length greater than the capillary channel 18, allowing the lance 24 to extend past both the first and the second ends 20, 22 of the capillary channel 18. Once the lance 24 has punctured the skin, the user pulls lance 24 out of the skin and up the capillary channel 18, at least past the reaction area 26. Blood is drawn into the capillary channel 18 via capillary action. The reagent 19 in the capillary channel 18 reacts with the blood to create a reaction that can be measured as discussed above. In some embodiments, the capillary channel 18 includes stops (not shown) that prevent the lance 24 from being completely pulled out of the capillary channel 18. In these embodiments, it is only necessary to pull the lance past the location of the reagent 19.

The fluid collection apparatus 10 as described provides the advantage of placing the harvesting or collection point of the sensor at the same location as the puncture wound from the lance 24. This eliminates the need to move the fluid collection apparatus 10 around after drawing blood in order to harvest the blood. The device 10 is easier to use, because the users will not have to manually manipulate the sensor after the puncture by trying to place the sensor at the precise location of the puncture.

While the present invention has been described with reference to one or more particular embodiments, those skilled in the art will recognize that many changes may be made thereto without departing from the scope of the present invention.

## Claims

1. A fluid collection apparatus (10) to test a concentration of an analyte in a fluid, comprising:
a lid (12);
a base (14) having a plane;
a spacer (16a, 16b) disposed between said lid (12) and said base (14), said spacer (16a, 16b) including a capillary channel (18) having an opening (22) for receiving the fluid; and
a lance (24) disposed between said lid (12) and said base (14) and
said lance (24) being moveable relative to said base (14) and parallel to said plane of said base (14) between a first position and a second position,
in said second position an end of said lance (24) extending beyond said lid (12) and said base (14) for puncturing and said end of the lance (24) being adjacent said opening (22) for receiving the fluid of the capillary channel (18), wherein said capillary channel (18) includes a reaction area (26) for containing a reagent (19)
**characterized in that** said lance (24) is disposed in said capillary channel (18) and said capillary channel (18) further includes stops that prevents said lance (24) from being completely pulled out of said capillary channel 18 when said lance 24 is being moved from the second position and up said capillary channel (18) at least past the reaction area (26).

2. The fluid collection apparatus (10) of claim 1, wherein said capillary channel (18) is elongated and said lance (24) is disposed in said elongated capillary channel (18), such that said lance (24) is moveable along the length of said capillary channel (18).

3. The fluid collection apparatus (10) of claim 1 or 2, wherein said capillary channel (18) has a length of approximately 0.508 (0.020) to approximately 1.016 mm (0.040 inches).

4. The fluid collection apparatus (10) of one of the claims 1 to 34, wherein said detection area (26) has an area of approximately 0.45 (0.7 x 10-³) to 6.45 mm² (10 x 10⁻³ inches squared).

5. The fluid collection apparatus (10) of one of the claims 1 to 4, wherein said capillary channel (30) has a width of approximately 0.15 (0.006) to approximately 0.3 mm (0.012 inches).

6. The fluid collection apparatus (10) of one of the claims 1 to 5, wherein said lance (24) has a diameter of approximately 0.13 mm (0.005) to approximately 0.28 mm (0.011 inches).

7. The fluid collection apparatus (10) of one of the claims 1 to 6, further comprising a reagent (19) disposed in said capillary channel (18) and adapted to produce a reaction indicative of the concentration of the analyte in the fluid.

8. The fluid collection apparatus (10) of claim 7, wherein the reagent (19) is adapted to produce a colorimetric reaction.

9. The fluid collection apparatus (10) of claim 8, in combination with a colorimetric test device.

10. The fluid collection apparatus (10) of claim 9, wherein the reagent (19) is adapted to produce an electrochemical reaction.

11. The fluid collection apparatus (10) of claim 10, in combination with an electrochemical test device.

12. The fluid collection apparatus (10) of one of the claims 1 to 11, wherein the analyte is glucose.

13. The fluid collection apparatus (10) of claim 12, in combination with a test device adapted to measure the concentration of glucose in blood.

14. The fluid collection apparatus (10) of one of the claims 1 to 13, in combination with a test device adapted to measure the absorption of infrared light by the fluid.

## Patentansprüche

1. Fluidauffangvorrichtung (10) zum Testen einer Konzentration eines Analyten in einem Fluid, Folgendes umfassend:
einen Deckel (12);
eine Basis (14), die eine Ebene aufweist;
ein Abstandsstück (16a, 16b), das zwischen dem Deckel (12) und der Basis (14) angeordnet ist, wobei das Abstandsstück (16a, 16b) ein Kapillarröhrchen (18) mit einer Öffnung (22) zur Aufnahme des Fluids umfasst; und
eine Lanzette (24), die zwischen dem Deckel (12) und
der Basis (14) angeordnet ist, und
wobei die Lanzette (24) relativ zur Basis (14) und
parallel zu der Ebene der Basis (14) zwischen einer ersten Position und einer zweiten Position bewegbar ist,
wobei sich ein Ende der Lanzette (24) in der zweiten Position zum Punktieren über den Deckel (12) und die Basis (14) hinaus erstreckt und das Ende der Lanzette (24) zum Aufnehmen des Fluids des Kapillarröhrchens (18) angrenzend an die Öffnung (22) angeordnet ist, wobei das Kapillarröhrchen (18) einen Reaktionsbereich (26) zum Enthalten eines Reagens (19) enthält, **dadurch gekennzeichnet, dass** die Lanzette (24) in dem Kapillarröhrchen (18) angeordnet ist und das Kapillarröhrchen (18) ferner Anschläge enthält, die verhindern, dass die Lanzette (24) vollständig aus dem Kapillarröhrchen (18) gezogen wird, wenn die Lanzette (24) aus der zweiten Position und das Kapillarröhrchen (18) hinauf zumindest an dem Reaktionsbereich (26) vorbei bewegt wird.

2. Fluidauffangvorrichtung (10) nach Anspruch 1, wobei das Kapillarröhrchen (18) länglich ist und die Lanzette (24) in dem länglichen Kapillarröhrchen (18) derart angeordnet ist, dass die Lanzette (24) entlang der Länge des Kapillarröhrchens (18) bewegbar ist.

3. Fluidauffangvorrichtung (10) nach Anspruch 1 oder 2, wobei das Kapillarröhrchen (18) eine Länge von ungefähr 0,508 mm (0,020 Zoll) bis ungefähr 1,016 mm (0,040 Zoll) aufweist.

4. Fluidauffangvorrichtung (10) nach einem der Ansprüche 1 bis 3, wobei der Erkennungsbereich (26) eine Fläche von ungefähr 0,45 mm² (0,7 x 10⁻³ Quadratzoll) bis 6,45 mm² (10 x 10-³ Quadratzoll) aufweist.

5. Fluidauffangvorrichtung (10) nach einem der Ansprüche 1 bis 4, wobei das Kapillarröhrchen (30) eine Breite von ungefähr 0,15 mm (0,006 Zoll) bis ungefähr 0,3 mm (0,012 Zoll) aufweist.

6. Fluidauffangvorrichtung (10) nach einem der Ansprüche 1 bis 5, wobei die Lanzette (24) einen Durchmesser von ungefähr 0,13 mm (0,005 Zoll) bis ungefähr 0,28 mm (0,011 Zoll) aufweist.

7. Fluidauffangvorrichtung (10) nach einem der Ansprüche 1 bis 6, ferner umfassend ein Reagens (19), das in dem Kapillarröhrchen (18) angeordnet ist und dazu geeignet ist, eine Reaktion hervorzurufen, die die Konzentration des Analyten im Fluid anzeigt.

8. Fluidauffangvorrichtung (10) nach Anspruch 7, wobei das Reagens (19) geeignet ist, eine kolorimetrische Reaktion hervorzurufen.

9. Fluidauffangvorrichtung (10) nach Anspruch 8, in Kombination mit einer kolorimetrischen Testvorrichtung.

10. Fluidauffangvorrichtung (10) nach Anspruch 9, wobei das Reagens (19) dazu geeignet ist, eine elektrochemische Reaktion hervorzurufen.

11. Fluidauffangvorrichtung (10) nach Anspruch 10, in Kombination mit einer elektrochemischen Testvorrichtung.

12. Fluidauffangvorrichtung (10) nach einem der Ansprüche 1 bis 11, wobei der Analyt Glucose ist.

13. Fluidauffangvorrichtung (10) nach Anspruch 12, in Kombination mit einer Testvorrichtung, die dazu geeignet ist, die Konzentration von Glucose im Blut zu messen.

14. Fluidauffangvorrichtung (10) nach einem der Ansprüche 1 bis 13, in Kombination mit einer Testvorrichtung, die dazu geeignet ist, die Absorption von Infrarotlicht durch das Fluid zu messen.

## Revendications

1. Appareil collecteur de fluide (10) pour tester une concentration d'un analyte dans un fluide, comprenant:
- un couvercle (12);
- une base (14) comportant un plan;
- un élément intercalaire (16a, 16b) disposé entre ledit couvercle (12) et ladite base (14), ledit élément intercalaire (16a, 16b) comprenant un canal capillaire (18) ayant un orifice (22) pour recevoir le fluide; et
- une lancette (24) disposée entre ledit couvercle (12) et ladite base (14), et
ladite lancette (24) est mobile par rapport à ladite base (14) et parallèle audit plan de ladite base (14) entre une première position et une seconde position, dans ladite seconde position, une extrémité de ladite lancette (24) s'étend au-delà dudit couvercle (12) et de ladite base (14) à des fins de perforation, et ladite extrémité de la lancette (24) est adjacente audit orifice (22) pour recevoir le fluide du canal capillaire (18), dans lequel ledit canal capillaire (18) comprend une zone de réaction (26) pour contenir un réactif (19),
**caractérisé en ce que** ladite lancette (24) est disposée dans ledit canal capillaire (18), et
ledit canal capillaire (18) comprend en outre des arrêts qui empêchent ladite lancette (24) d'être complètement tirée hors dudit canal capillaire (18) lorsque ladite lancette (24) est déplacée à partir de la seconde position et vers le haut dudit canal capillaire (18) au-moins au-delà de la zone de réaction (26).

2. Appareil collecteur de fluide (10) selon la revendication 1, dans lequel ledit canal capillaire (18) est allongé et ladite lancette (24) est disposée dans ledit canal capillaire allongé (18), de telle sorte que ladite lancette (24) soit mobile le long de la longueur dudit canal capillaire (18).

3. Appareil collecteur de fluide (10) selon la revendication 1 ou 2, dans lequel ledit canal capillaire (18) présente une longueur d'environ 0,508 mm (0,020 pouce) à environ 1,016 mm (0,040 pouce) .

4. Appareil collecteur de fluide (10) selon l'une des revendications 1 à 3, dans lequel ladite zone de détection (26) présente une surface d'environ 0,45 mm² (0,7 x 10-³ pouces carrés) à environ 6,45 mm² (10 x 10-³ pouces carrés).

5. Appareil collecteur de fluide (10) selon l'une des revendications 1 à 4, dans lequel ledit canal capillaire (30) présente une largeur d'environ 0,15 mm (0,006 pouce) à environ 0,3 mm (0,012 pouce).

6. Appareil collecteur de fluide (10) selon l'une des revendications 1 à 5, dans lequel ladite lancette (24) présente un diamètre d'environ 0,13 mm (0,005 pouce) à environ 0,28 mm (0,011 pouce).

7. Appareil collecteur de fluide (10) selon l'une des revendications 1 à 6, comprenant en outre un réactif (19) disposé dans ledit canal capillaire (18) et adapté pour provoquer une réaction indicative de la concentration de l'analyte dans le fluide.

8. Appareil collecteur de fluide (10) selon la revendication 7, dans lequel le réactif (19) est adapté pour provoquer une réaction colorimétrique.

9. Appareil collecteur de fluide (10) selon la revendication 8, en combinaison avec un dispositif de test colorimétrique.

10. Appareil collecteur de fluide (10) selon la revendication 9, dans lequel le réactif (19) est adapté pour provoquer une réaction électrochimique.

11. Appareil collecteur de fluide (10) selon la revendication 10, en combinaison avec un dispositif de test électrochimique.

12. Appareil collecteur de fluide (10) selon l'une des revendications 1 à 11, dans lequel l'analyte est le glucose.

13. Appareil collecteur de fluide (10) selon la revendication 12, en combinaison avec un dispositif de test adapté pour mesurer la concentration de glucose dans le sang.

14. Appareil collecteur de fluide (10) selon l'une des revendications 1 à 13, en combinaison avec un dispositif de test adapté pour mesurer l'absorption de lumière infrarouge par le fluide.
